(19) Europäisches Patentamt
European Patent Office
Office européen des brevets

(11) Veröffentlichungsnummer: **0 189 883**
**A1**

(12) **EUROPÄISCHE PATENTANMELDUNG**

(21) Anmeldenummer: **86101054.4**

(22) Anmeldetag: **27.01.86**

(51) Int. Cl.⁴: **C 07 D 471/04**
A 61 K 31/505
//(C07D471/04, 239:00, 221:00)

(30) Priorität: **28.01.85 DE 3502742**

(43) Veröffentlichungstag der Anmeldung:
**06.08.86 Patentblatt 86/32**

(84) Benannte Vertragsstaaten:
**AT CH DE FR GB IT LI**

(71) Anmelder: **GÖDECKE AKTIENGESELLSCHAFT**
**Salzufer 16**
**D-1000 Berlin 10(DE)**

(72) Erfinder: **Kleinschroth, Jürgen, Dr.**
**Freiburger Strasse 13**
**D-7809 Denzlingen(DE)**

(72) Erfinder: **Mannhardt, Karl, Dr.**
**Pfauenstrasse 14**
**D-7807 Elzach-Oberprechtal(DE)**

(72) Erfinder: **Hartenstein, Johannes, Dr.**
**Fohrenbühl 23**
**D-7801 Stegen-Wittental(DE)**

(72) Erfinder: **Osswald, Hartmut, Prof. Dr.**
**Kiefernweg 1**
**D-7808 Waldkirch 2(DE)**

(72) Erfinder: **Wagner, Bernd, Dr.**
**Am Lossele 5**
**D-7809 Denzlingen(DE)**

(54) **5-Oxo-pyrido(4,3-d)pyrimidin-Derivate, Verfahren zu deren Herstellung und diese enthaltende Arzneimittel.**

(57) Die Erfindung betrifft neue 5-Oxo-pyrido[4,3-d]pyrimidin-Derivate der allgemeinen Formel 1a,

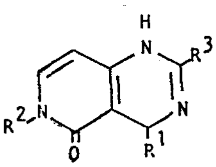

(Ia)

bzw. deren tautomere Formen der allgemeinen Formeln 1b und 1c

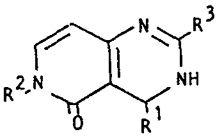

(Ib)

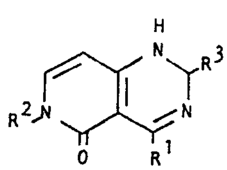

(Ic)

oder aus den Gleichgewichten dieser Formen bestehende Verbindungen, in welchen

$R^1$ einen unsubstituierten oder substituierten Phenylrest

$R^2$ Wasserstoff, eine Alkyl-, Alkoxyalkyl- oder eine substituierte oder unsubstituierte Aminoalkylgruppe mit bis zu 10 Kohlenstoffatomen und

$R^3$ eine geradkettige oder verzweigte Alkylgruppe mit bis zu 4 Kohlenstoffatomen oder einen unsubstituierten oder substituierten Phenylrest bedeutet,

und deren pharmakologisch unbedenkliche Salze.

Die Erfindung betrifft weiterhin ein chemisch eigenartiges Herstellungsverfahren und Arzneimittel welche die neuen Verbindungen Ia, b, c als Wirkstoff zur Bekämpfung cerebraler, cardialer oder peripherer Gefäßerkrankungen oder von stenotischen Erscheinungen enthalten.

EP 0 189 883 A1

Croydon Printing Company Ltd

# BESCHREIBUNG

Die Erfindung betrifft neue 5-Oxo-pyrido[4,3-d]pyrimidin-Derivate der allgemeinen Formel Ia,

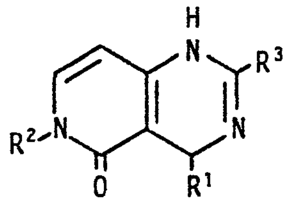

(Ia)

bzw. deren tautomere Formen der allgemeinen Formeln Ib und Ic

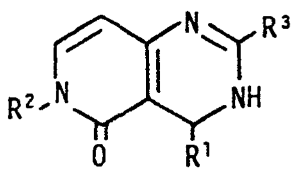

(Ib)

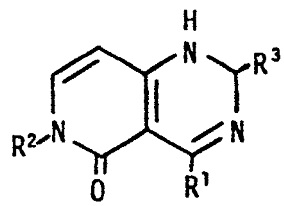

(Ic)

oder aus den Gleichgewichten dieser Formen bestehende Verbindungen, in welchen

$R^1$ einen unsubstituierten oder substituierten Phenylrest

$R^2$ Wasserstoff, eine Alkyl-, Alkoxyalkyl- oder eine substituierte oder unsubstituierte Aminoalkylgruppe mit bis zu 10 Kohlenstoffatomen und

$R^3$ eine geradkettige oder verzweigte Alkylgruppe mit bis zu 4 Kohlenstoffatomen oder einen unsubstituierten oder substituierten Phenylrest bedeutet,

sowie gegebenenfalls deren pharmakologisch unbedenkliche Salze.

Bevorzugt werden Verbindungen der Formeln Ia, Ib, Ic in welchen

$R^1$ einen unsubstituierten oder in 2- und/oder 3- oder 6-Position substituierten Phenylrest

$R^2$ eine $C_1$ - $C_6$ Alkylgruppe, eine $C_1$ - $C_8$ Alkoxyalkylgruppe oder eine $C_1$ - $C_8$ Aminoalkylgruppe und

$R^3$ einen $C_1$ - $C_6$ Alkyl- oder einen Phenylrest

bedeuten.

Besonders bevorzugt werden 5-Oxo-pyrido [4,3-d] pyrimidin-Derivate der allgemeinen Formeln Ia, bzw. Ib und Ic in welchen

$R^1$ einen unsubstituierten oder einen vorzugsweise in 2- oder 3-Position durch Halogen, Nitro, Methyl, Methoxy, Difluormethoxy, Trifluormethoxy, Dimethyl- oder Diethylamino, Methylthio, oder Trifluormethyl substituierten Phenylrest oder einen vorzugsweise in 2,3- durch Methoxy oder Methylendioxy oder in 2,3- oder 2,6-Position durch Halogenatome, die gleich oder verschieden sein können, disubstituierten Phenylrest,

$R^2$ einen Methyl-, Ethyl-, n-Propyl-, Isopropyl-, n-Butyl-, Isobutyl- oder tert. Butylrest, eine Alkoxyalkylgruppe der allgemeinen Formel II

$$-(CH_2)_n-O-R^4 \qquad (II)$$

oder eine Aminoalkylgruppe der allgemeinen Formel III

$$-(CH_2)_n-N\begin{matrix}\diagup R^5\\ \diagdown R^6\end{matrix} \qquad (III)$$

worin $R^4$ eine niedere geradkettige oder verzweigte Alkylgruppe darstellt und $R^5$ und $R^6$ gleich oder verschieden sein können und eine geradkettige oder verzweigte niedere Alkylgruppe darstellen, oder gemeinsam eine niedere Alkylengruppe bilden und n gleich 2 oder 3 ist und

$R^3$ einen Methyl-, Ethyl-, n-Propyl-, Isopropyl- oder Phenylrest bedeuten.

Die Erfindung betrifft weiterhin ein chemisch eigenartiges Verfahren zur Herstellung von 5-Oxo-pyrido[4,3-d]pyrimidin-Derivaten der allgemeinen Formeln Ia,b,c über Zwischenprodukte der allgemeinen Formeln IVa.

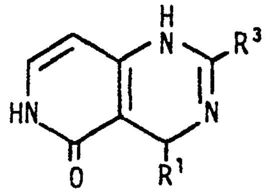

(IVa)

bzw. IVb und IVc

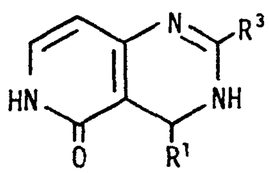

(IVb)

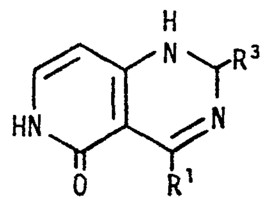

(IVc)

oder aus den Gleichgewichten dieser Formen bestehenden Verbindungen, in welchen $R^1$ und $R^3$ die oben angegebene Bedeutung haben.
Die Verbindungen der allgemeinen Formeln Ia bzw. Ib und Ic, erhält man dadurch, daß man Verbindungen der allgemeinen Formeln IVa bzw. IVb und IVc in an sich bekannter Weise alkyliert, aminoalkyliert oder alkoxyalkyliert.

Die Verbindungen der allgemeinen Formeln IVa bzw. IVb und IVc werden hergestellt, in dem man Dihydropyrimidine der allgemeinen Formeln Va

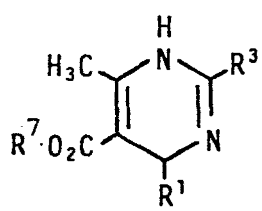

(Va)

bzw. Vb und Vc

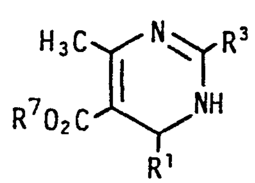

(Vb)

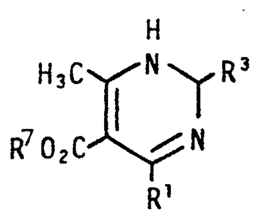

(Vc)

0189883

oder aus den Gleichgewichten dieser Formen bestehende Verbindungen, in welchen $R^1$ und $R^3$ die oben angegebene Bedeutung besitzen und $R^7$ eine Methyl- oder Ethylgruppe bedeutet, in Gegenwart einer Base mit s-Triazin umsetzt. Diese Reaktion ist bisher nicht bekannt und vom Verlauf her nicht vorhersehbar gewesen.

Die Herstellung der Verbindungen der allgemeinen Formeln Ia bzw. Ib und Ic erfolgt in an sich bekannter Weise durch Alkylierung bzw. Amino- oder Alkoxyalkylierung der Verbindungen der allgemeinen Formeln IVa bzw. IVb und IVc mit Verbindungen der allgemeinen Formel VI

$$X\text{-}R^2 \qquad \text{(VI)}$$

in welcher $R^2$ die oben genannte Bedeutung hat und X für Halogen, insbesondere Chlor, Brom oder Iod steht, vorzugsweise in Gegenwart eines Halogenwasserstoffakzeptors, z.B. Triethylamin oder Pyridin.

Die Verbindungen der allgemeinen Formeln Va bzw. Vb und Vc sind literaturbekannt (DE-OS 32 34 684) oder können in analoger Weise hergestellt werden.

Zur Durchführung der Reaktion wird das Dihydropyrimidinderivat mit s-Triazin in einem inerten organischen Lösungsmittel in Gegenwart starker Basen wie z.B. Alkalialkoholaten oder Natriumhydrid auf Temperaturen von 50-160°C, vorzugsweise jedoch 100-150°C, erhitzt. Als Solventien eignen sich hier vor allem polare Lösungsmittel wie Dimethylsulfoxid, Dimethylformamid oder Ethylenglykoldimethylether.

Die Alkylierung, Amino- und Alkoxyalkylierung der Verbindungen der allgemeinen Formeln IVa bzw. IVb und IVc erfolgt nach allgemein bekannten Methoden, vorzugsweise unter Verwendung eines Halogenwasserstoffakzeptors. Die Reaktion verläuft bei der Wahl geeigneter Reaktionsbedingungen mit hoher Regioselektivität. Die zu erwartenden O-Alkylierungsprodukte werden überraschend nur in geringen Mengen gebildet. Die Trennung bzw. Reinigung der Produkte erfolgt mittels Chromatographie und/oder Kristallisation.

In Abhängigkeit von den Substituenten $R^1$ und $R^3$ weisen Verbindungen der allgemeinen Formeln Ia bzw. Ib und Ic am Pyrimidinring mehr oder minder basischen Charakter auf und werden deshalb ebenso wie Verbindungen der allgemeinen Formeln Ia bzw. Ib und Ic, die für $R^2$ eine substituierte oder unsubstituierte Aminoalkylgruppe aufweisen, zum Zwecke der Reinigung und aus galenischen Gründen bevorzugt in kristalline, pharmakologisch verträgliche Salze übergeführt. Diese werden in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure oder Bernsteinsäure in Frage.

Da die erfindungsgemäßen Verbindungen der allgemeinen Formeln Ia bzw. Ib und Ic entweder an C-2 oder an C-4 ein chirales Zentrum aufweisen, können sie entweder als racemische Gemische oder in Form der Enantiomeren vorliegen.

Die Verbindungen der allgemeinen Formeln Ia bzw. Ib und Ic zeigen als Calciumantagonisten gefäßspasmolytische, vasodilatatorische und antihypertensive Wirkungen.

Aufgrund ihrer gefäßspasmolytischen Wirkungen sind sie vor allem bei cerebralen, cardialen und peripheren Gefäßerkrankungen wie myokardialer Ischämie, bei cerebralem Infarkt, pulmonalen Thrombosen und bei Arteriosklerose und anderen stenotischen Erscheinungen indiziert. Die 5-Oxo-pyrido[4,3-d]pyrimidin-Derivate der vorliegenden Erfindung sind daher wertvolle Mittel für die Bekämpfung der Herz-Kreislauf-Mortalität. Ein weiterer Gegenstand der vorliegenden Erfindung ist daher die Verwendung der 5-Oxo-pyrido[4,3-d]pyrimidine der allgemeinen Formeln Ia bzw. Ib und Ic bei der Bekämpfung von Gefäßerkrankungen.

Die erfindungsgemäßen Verbindungen der allgemeinen Formeln Ia bzw. Ib und Ic können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citrat-Puffer, Ethanol, Komplexbildner (wie Äthylendiamin-tetraessigsäure und deren nichttoxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süßstoffe enthalten.

Die enteral verabreichten Einzeldosen liegen im Bereich von etwa 5 bis 250 mg, vorzugsweise 10 bis 100 mg. Parenteral werden etwa 1 bis 20 mg gegeben.

Die folgenden Beispiele sollen die Erfindung näher erläutern:

0189883

## Beispiel 1

(±)-4-(3-Chlorphenyl)-1,4,5,6-tetrahydro-5-oxo-2-phenyl-pyrido-[4,3-d]pyrimidin

Zu einer gerührten Suspension von 6,0 g (0,20 Mol) Natriumhydrid (80%ig in Öl) in 100 ml trockenem Dimethylformamid wird unter Stickstoffatmosphäre eine Lösung von 46,4 g (0,14 Mol) (±)-4-(3-Chlorphenyl)-1,4-dihydro-6-methyl-2-phenyl-pyrimidin-5-carbonsäuremethylester +) in 300 ml Dimethylformamid getropft. Bei Nachlassen der Gasentwicklung wird noch 30 Minuten bei Raumtemperatur gerührt; anschließend werden 11,3 g (0,14 Mol) s-Triazin in 200 ml Dimethylformamid zugetropft. Die Reaktionsmischung wird 16 Stunden auf 100 bis 110°C erhitzt und nach dem Abkühlen im Vakuum eingeengt. Der dunkle Rückstand wird mit 1 Liter Aceton im Ultraschallbad behandelt und vom Ungelösten filtriert. Die Acetonlösung wird im Vakuum eingedampft und der Rückstand an Kieselgel mit Dichlormethan/Methanol 9:1 chromatographiert. Die Fraktion mit dem $R_f$ 0,45 wird isoliert und aus Methanol umkristallisiert.

Man erhält (±)-4-(3-Chlorphenyl)-1,4,5,6-tetrahydro-5-oxo-2-phenyl-pyrido[4,3-d]pyrimidin in Form hellbeiger Kristalle vom Schmp. 172-173°C.

+) Lit.: DE-OS 32 34 684

In analoger Weise werden die folgenden Verbindungen erhalten:

(±)-1,4,5,6-Tetrahydro-4-(3-nitrophenyl)-5-oxo-2-phenyl-pyrido[4,3-d]pyrimidin (1.a)

Schmp. 206-207°C aus Methylenchlorid

(±)-1,4,5,6-Tetrahydro-2-methyl-5-oxo-4-phenyl-pyrido-[4,3-d]pyrimidin (1.b)

Schmp. 263-264°C aus Ethylacetat/Methanol

(±)-1,4,5,6-Tetrahydro-2-methyl-5-oxo-4-(2-trifluormethyl-phenyl)-pyrido[4,3-d]pyrimidin (1.c)

Schmp. 226-228°C aus Ethylacetat/Methanol

(±)-1,4,5,6-Tetrahydro-2-methyl-4-(2-nitrophenyl)-5-oxo-pyrido[4,3-d]pyrimidin (1.d)

(±)-1,4,5,6-Tetrahydro-2-(3-nitrophenyl)-5-oxo-4-(2-trifluormethylphenyl)-pyrido[4,3-d] pyrimidin (1.e)

## Beispiel 2

(±)-1,4,5,6-Tetrahydro-6-isopropyl-2-methyl-5-oxo-4-phenyl-pyrido-[4,3-d]pyrimidin · Hydrochlorid

Zu einer gerührten Suspension von 1,24 g (41,3 mMol) Natriumhydrid (80%ig in Öl) in 50 ml trockenem Dimethylformamid werden 8,97 g (37,5 mMol) (±)-1,4,5,6-Tetrahydro-2-methyl-5-oxo-4-phenyl-pyrido[4,3-d]pyrimidin portionsweise fest zugegeben. Bei Nachlassen der Gasentwicklung wird 30 Minuten bei Raumtemperatur gerührt; anschließend werden 9,56 g (56,2 mMol) Isopropyliodid in 20 ml Dimethylformamid zugetropft.

Es wird 72 Stunden bei Raumtemperatur gerührt, das Lösungsmittel im Vakuum abgezogen und der Rückstand mit 100 ml Wasser verrührt. Das Wasser wird abdekantiert, der Rückstand in Dichlormethan gelöst und die Lösung erneut mit Wasser gewaschen. Die Dichlormethanlösung wird über Natriumsulfat getrocknet und im Vakuum eingedampft. Der Rückstand wird an Kieselgel mit Ethylacetat/Methanol, $NH_3$ ges. 95:5 chromatographiert.

Die Fraktion mit dem $R_f$ 0,1 wird in Ether/Ethylacetat gelöst und mit der berechneten Menge Chlorwasserstoff in Ethylacetat in das Hydrochlorid übergeführt und dieses aus Isopropanol/Diisopropylether umkristallisiert.

Man erhält das Hydrochlorid des (±)-1,4,5,6-Tetrahydro-6-isopropyl-2-methyl- 5-oxo-4-phenyl-pyrido[4,3-d]pyrimidin in Form farbloser Kristalle vom Schmp. 296-297°C (Z).

Als weiteres Produkt wird bei der obigen Chromatographie (±)-1,4-Dihydro-5-isopropoxy-2-methyl-4-phenyl-pyrido-[4,3-d]pyrimidin ($R_f$ 0,3) isoliert.

I. Patentansprüche für die Vertragsstaaten CH, DE, FR, GB, IT

1.) 5-Oxo-pyrido[4,3-d]pyrimidin-Derivate der allgemeinen Formel Ia,

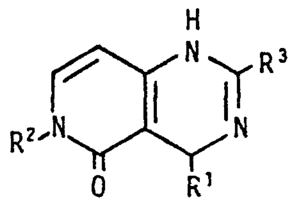

(Ia)

bzw. deren tautomere Formen der allgemeinen Formeln Ib und Ic

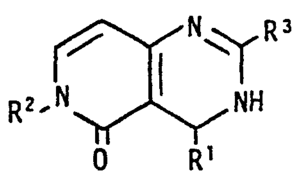

(Ib)

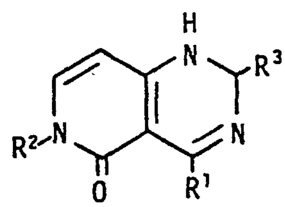

(Ic)

oder aus den Gleichgewichten dieser Formen bestehende Verbindungen, in welchen

$R^1$ einen unsubstituierten oder substituierten Phenylrest

$R^2$ Wasserstoff, eine Alkyl-, Alkoxyalkyl- oder eine substituierte oder unsubstituierte Aminoalkylgruppe mit bis zu 10 Kohlenstoffatomen und

$R^3$ eine geradkettige oder verzweigte Alkylgruppe mit bis zu 4 Kohlenstoffatomen oder einen unsubstituierten oder substituierten Phenylrest bedeutet,

sowie gegebenenfalls deren pharmakologisch unbedenkliche Salze.

2.) 5-Oxo-pyrido[4,3-d]pyrimidin-Derivate gemäß Anspruch 1, worin

$R^1$ einen unsubstituierten oder in 2- und/oder 3-Position substituierten Phenylrest

$R^2$ eine $C_1$-$C_6$ Alkylgruppe, eine $C_1$-$C_8$ Alkoxyalkylgruppe oder eine $C_1$-$C_8$ Aminoalkylgruppe und

$R^3$ einen $C_1$-$C_6$ Alkyl- oder einen Phenylrest

bedeuten.

3.) 5-Oxo-pyrido[4,3-d]pyrimidin-Derivate nach Anspruch 1 oder 2 der allgemeinen Formeln Ia, bzw. Ib und Ic in welchen

$R^1$ einen unsubstituierten oder einen durch Halogen, Nitro, Methyl, Methoxy, Difluormethoxy, Trifluormethoxy, Dimethylamino, Diethylamino, Methylthio, oder Trifluormethyl substituierten Phenylrest oder einen durch Methoxy, Methylendioxy oder Halogenatome, die gleich oder verschieden sein können, disubstituierten Phenylrest,

$R^2$ einen Methyl-, Ethyl-, n-Propyl-, Isopropyl-, n-Butyl-, Isobutyl- oder tert. Butylrest, eine Alkoxyalkylgruppe der allgemeinen Formel II

$$-(CH_2)_n-O-R^4 \qquad (II)$$

oder eine Aminoalkylgruppe der allgemeinen Formel III

$$-(CH_2)_n-N\begin{matrix} \diagup R^5 \\ \diagdown R^6 \end{matrix} \qquad (III)$$

worin $R^4$ eine niedere geradkettige oder verzweigte Alkylgruppe darstellt und $R^5$ und $R^6$ gleich oder verschieden sein können und eine geradkettige oder verzweigte niedere Alkylgruppe darstellen, oder gemeinsam eine niedere Alkylengruppe bilden und n gleich 2 oder 3 ist und

$R^3$ einen Methyl-, Ethyl-, n-Propyl-, Isopropyl- oder Phenylrest

bedeuten.

0189883

4.) Verfahren zur Herstellung von 5-Oxo-pyrido[4,3-d]pyrimidin-Derivaten gemäß Anspruch 1 bis 3, dadurch gekennzeichnet, daß ein Dihydropyrimidin der allgemeinen Formeln Va, b, c

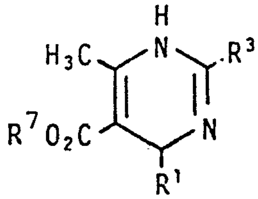 (Va) 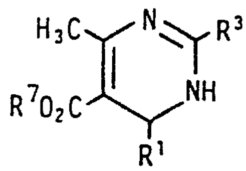 (Vb) 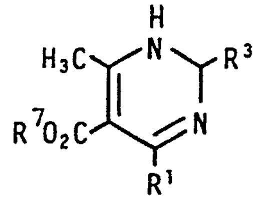 (Vc)

in welchen
die Reste $R^1$ und $R^3$ die in den jeweiligen Ansprüchen 1 bis 3 angegebene Bedeutung haben und $R^7$ eine Methyl- oder Ethylgruppe bedeutet

oder aus den Gleichgewichten dieser Formen bestehende Verbindungen in Gegenwart einer Base mit s-Triazin umsetzt, gegebenenfalls anschließend zur Einführung des Restes $R^2$ in an sich bekannter Weise alkyliert, aminoalkyliert oder alkoxyalkyliert und gewünschtenfalls mittels einer Säure in ein pharmakologisch verträgliches Salz überführt.

5.) Arzneimittel enthaltend eine Verbindung nach Anspruch 1 bis 3 zur Bekämpfung cerebraler, cardialer oder peripherer Gefäßerkrankungen oder von stenotischen Erscheinungen.

## I. Patentansprüche für Vertragsstaat AT

1.) Verfahren zur Herstellung von 5-Oxo-pyrido[4,3-d]pyrimidin-Derivaten der allgemeinen Formal Ia,

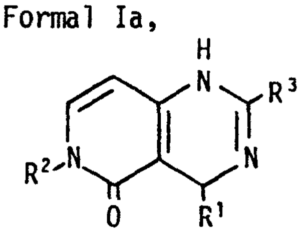

(Ia)

bzw. von deren tautomeren Formen der allgemeinen Formeln Ib und Ic

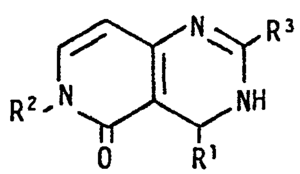

(Ib)

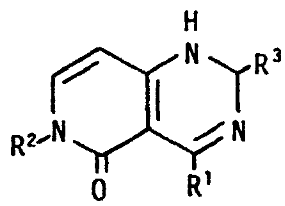

(Ic)

oder von aus den Gleichgewichten dieser Formen bestehenden Verbindungen in welchen

$R^1$ einen unsubstituierten oder substituierten Phenylrest

$R^2$ Wasserstoff, eine Alkyl-, Alkoxyalkyl- oder eine substituierte oder unsubstituierte Aminoalkylgruppe mit bis zu 10 Kohlenstoffatomen und

$R^3$ eine geradkettige oder verzweigte Alkylgruppe mit bis zu 4 Kohlenstoffatomen odereinen unsubstituierten oder substituierten Phenylrest bedeutet,

sowie gegebenenfalls von deren pharmakologisch unbedenklichen Salzen, dadurch gekennzeichnet, daß ein Dihydropyrimidin der allgemeinen Formeln Va, b, c

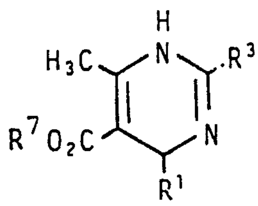 (Va) 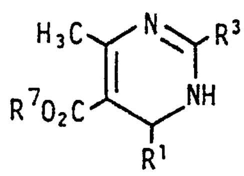 (Vb) 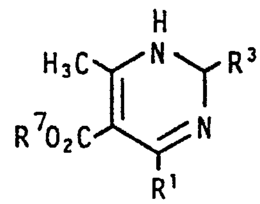 (Vc)

in welchen
die Reste $R^1$ und $R^3$ die oben angegebene Bedeutung haben und $R^7$ eine Methyl- oder Ethylgruppe bedeutet

oder aus den Gleichgewichten dieser Formen bestehende Verbindungen in Gegenwart einer Base mit s-Triazin umsetzt, gegebenenfalls anschließend zur Einführung des Restes $R^2$ in an sich bekannter Weise alkyliert, aminoalkyliert oder alkoxyalkyliert und gewünschtenfalls mittels einer Säure in ein pharmakologisch verträgliches Salz überführt wird.

2.) Verfahren gemäß Anspruch 1, dadurch gekennzeichnet, daß

$R^1$ einen unsubstituierten oder in 2- und/oder 3-Position substituierten Phenylrest

$R^2$ eine $C_1$-$C_6$ Alkylgruppe, eine $C_1$-$C_8$ Alkoxyalkylgruppe oder eine $C_1$-$C_8$ Aminoalkylgruppe und

$R^3$ einen $C_1$-$C_6$ Alkyl- oder einen Phenylrest

bedeuten.

3.) Verfahren nach Anspruch 1 und 2, dadurch gekennzeichnet, daß

$R^1$ einen unsubstituierten oder einen durch Halogen, Nitro, Methyl, Methoxy, Difluormethoxy, Trifluormethoxy, Dimethylamino, Diethylamino, Methylthio, oder Trifluormethyl substituierten Phenylrest oder einen durch Methoxy, Methylendioxy oder Halogenatome, die gleich oder verschieden sein können, disubstituierten Phenylrest,

$R^2$ einen Methyl-, Ethyl-, n-Propyl-, Isopropyl-, n-Butyl-, Isobutyl- oder tert. Butylrest, eine Alkoxyalkylgruppe der allgemeinen Formel II

$$-(CH_2)_n-O-R^4 \qquad (II)$$

oder eine Aminoalkylgruppe der allgemeinen Formel III

$$-(CH_2)_n-N\begin{matrix} \diagup R^5 \\ \diagdown R^6 \end{matrix} \qquad (III)$$

worin $R^4$ eine niedere geradkettige oder verzweigte Alkylgruppe darstellt und $R^5$ und $R^6$ gleich oder verschieden sein können und eine geradkettige oder verzweigte niedere Alkylgruppe darstellen, oder gemeinsam eine niedere Alkylengruppe bilden und n gleich 2 oder 3 ist und

$R^3$ einen Methyl-, Ethyl-, n-Propyl-, Isopropyl- oder Phenylrest bedeuten.

Europäisches Patentamt

**0189883**

# EUROPÄISCHER RECHERCHENBERICHT

Nummer der Anmeldung

EP 86 10 1054

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | EP-A-0 103 796 (BAYER)<br>* Patentansprüche 1 und 10 * & DE - A - 3 234 684<br>----- | 1,5 | C 07 D 471/04<br>A 61 K 31/505//<br>(C 07 D 471/04<br>C 07 D 239:00<br>C 07 D 221:00) |
| | | | **RECHERCHIERTE SACHGEBIETE (Int. Cl.4)** |
| | | | C 07 D 471/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 09-05-1986 | ALFARO I. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82